(19)

Europäisches
Patentamt

European
Patent Office

Office européen
des brevets

(11) **EP 4 353 141 A1**

(12) **EUROPEAN PATENT APPLICATION**

(43) Date of publication:
**17.04.2024 Bulletin 2024/16**

(21) Application number: **23202681.5**

(22) Date of filing: **10.10.2023**

(51) International Patent Classification (IPC):
**A61B 3/107** *(2006.01)*    *A61B 3/00* *(2006.01)*
**A61B 3/10** *(2006.01)*

(52) Cooperative Patent Classification (CPC):
**A61B 3/107;** A61B 3/0058; A61B 3/101

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **12.10.2022 JP 2022164181**

(71) Applicant: **Tomey Corporation
Nagoya-shi, Aichi 451-0051 (JP)**

(72) Inventors:
• **TAKATA, Hideo
Nagoya-shi, Aichi 4510051 (JP)**
• **KATAOKA, Hisashi
Nagoya-shi, Aichi 4510051 (JP)**

(74) Representative: **Winter, Brandl - Partnerschaft
mbB
Alois-Steinecker-Straße 22
85354 Freising (DE)**

(54) **OPHTHALMIC DEVICE**

(57) An ophthalmic device disclosed herein may include: a projection unit configured to project pattern light onto a corneal surface of a subject eye; a first image capturing unit configured to capture a reflected image of the pattern light reflected from the corneal surface; a controller configured to cause the first image capturing unit to repeatedly capture a reflected image of the pattern light reflected from the corneal surface at predetermined time intervals; and an image processing unit configured to process captured images captured by the first image capturing unit. The pattern light may have a preset pattern formed of lines. The image processing unit may be further configured to calculate, for each of the captured images, a sum of absolute values of deformations in lines of a pattern image created by the pattern light in the captured image.

EP 4 353 141 A1

**Description**

Technical Field

The technique disclosed herein relates to an ophthalmic device, specifically to an ophthalmic device configured to capture reflected images of pattern light projected onto a corneal surface.

Background Art

For measurement of states of dry eyes, ophthalmic devices have been developed that are configured to project pattern light onto a corneal surface and capture reflected images of the pattern light reflected from the corneal surface (e.g., see Japanese Patent Application Publication No. 2004-321508). An ophthalmic device described in Japanese Patent Application Publication No. 2004-321508 projects ring-shaped pattern light onto a corneal surface, and observes, captures, and analyzes reflected images of the pattern light reflected from the corneal surface to measure the corneal surface condition and corneal shape. Whether the subject eye is a dry eye or not is determined by comparing chronological changes in the corneal shape from the start of measurement.

Summary

Ophthalmologists practice examinations following the dry eye practice guidelines (dry eye diagnosis flow) in order to evaluate dry eyes. Specifically, they stain a corneal surface with fluorescein, ask the subject to close the eyelids and then open them immediately thereafter, and measure a breakup time of the corneal staining. However, there are various types of dry eyes and it is impossible to identify the type of the dry eyes based only on the breakup time of corneal staining. Disclosed herein is an ophthalmic device configured to provide useful information for dry eye diagnosis.

An ophthalmic device disclosed herein may comprise: a projection unit configured to project pattern light onto a corneal surface of a subject eye; a first image capturing unit configured to capture a reflected image of the pattern light reflected from the corneal surface; a controller configured to cause the first image capturing unit to repeatedly capture a reflected image of the pattern light reflected from the corneal surface at predetermined time intervals; and an image processing unit configured to process captured images captured by the first image capturing unit. The pattern light may be light having a preset pattern formed of lines. The image processing unit may be further configured to calculate, for each of the captured images, a sum of absolute values of deformations in lines of a pattern image created by the pattern light in the captured image.

Conventional ophthalmic devices determine dry eye conditions by capturing an image immediately after eyelids open to use the image as a reference and compare this reference image with images captured thereafter. However, there are various types of dry eyes (e.g., aqueous deficient type dry eye, decreased wettability type dry eye, increased evaporation type dry eye, etc.), and for the decreased wettability type, deformations in a reflected image of pattern light in the image captured immediately after the eyelids open are the largest and deformations in reflected images in the subsequent images become smaller. Therefore, using the image captured immediately after the eyelids open as a reference may lead to an inaccurate assessment on changes in the tear film on the corneal surface. Contrary to this, in the ophthalmic device described above, a sum of absolute values of deformations in lines of a pattern image created by the pattern light is calculated for each of the captured images. That is, a sum of absolute values of deformations in lines of a pattern image is calculated for each of the captured images without using the image captured immediately after the eyelids open as a reference. Thus, changes in the tear film on the corneal surface can be accurately assessed.

The ophthalmic device disclosed herein may further comprise an output unit configured to chronologically output the sums of the absolute values of deformations from a time when capturing of the images is started or a time after a predetermined time period from the start of the capturing of the images. According to this configuration, a chronological change of the sums of the absolute values of deformations is output, and thus a chronological change of dry eye condition can be assessed.

In the ophthalmic device disclosed herein, the output unit may be further configured to output a chronological change in the sums of the absolute values of deformations over time. The chronological change may be a change in the sums of the absolute values of deformations over a predetermined time period from the time when the capturing of the images is started or the time after a predetermined time period from the start of the capturing of the images. This configuration allows for an assessment on whether deformations increase or decrease over time. Thus, useful information for determining a dry eye type can be provided.

The ophthalmic device disclosed herein may further comprise a second image capturing unit configured to capture an image of the subject eye while the pattern light is not projected; and a display unit configured to display the image of the subject eye. The display unit may be further configured to display a graph showing the chronological change in the sums of the absolute values of deformations together with the image of the subject eye. According to this configuration, the chronological change in the sums of the absolute values of deformations is displayed as a graph, and thus how they change can be easily understood.

In the ophthalmic device disclosed herein, the image processing unit may be further configured to identify, in each of the captured images, a variated site where the sum of the absolute values of deformations exceeds a predetermined value. The display unit may be further configured to display the variated site such that the variated site is superimposed on the image of the subject eye. According to this configuration, useful information for determining a dry eye type can be provided.

In the ophthalmic device disclosed herein, the display unit may be further configured to display a mark indicating a time when a captured image including the variated site was captured such that the mark is superimposed on the graph showing the chronological change in the sums of the absolute values of deformations. This configuration allows a time when the variated site where the pattern image has variated (i.e., where the tear film has changed) appears to be specified.

The ophthalmic device disclosed herein may further comprise an eyelids detection unit configured to detect opening of eyelids of the subject eye. The image processing unit may be further configured to correct the sums of the absolute values of deformations calculated for the captured images such that the sum of the absolute values of deformations calculated for a captured image captured immediately after the eyelids detection unit detected opening of the eyelids of the subject eye becomes a predetermined value. According to this configuration, it is possible to easily understand change(s) that occurred in the sums of the absolute values of deformations calculated for captured images captured after a first captured image captured immediately after the opening of the eyelids was detected as compared to the sum of the absolute values of deformations calculated for the first captured image.

Brief Description of Drawings

FIG. 1 shows a schematic diagram of an entire optical system of an ophthalmic device according to an embodiment.

FIG. 2 shows a block diagram showing a configuration of a control system of the ophthalmic device shown in FIG. 1.

FIG. 3 shows a flowchart for measuring a chronological change in a tear film on a corneal surface.

FIG. 4 shows an example of an image (original image) of reflected figure from a corneal surface.

FIG. 5 shows an example of a polar coordinate transformed image generated by performing polar coordinate transformation to the original image shown in FIG. 4.

FIG. 6 shows an example of a corrected image generated by correcting deformations in the polar coordinate transformed image shown in FIG. 5.

FIG. 7 shows an example of a superimposed image in which a variated site is superimposed on the original image.

FIG. 8 is an example of an image displayed on a display unit of the ophthalmic device according to the embodiment.

FIG. 9 shows an enlarged view of a major part of the image displayed on the display unit of the ophthalmic device according to the embodiment.

FIG. 10 shows another example of an image displayed on the display unit of the ophthalmic device according to the embodiment, where the image has been subjected to offset processing.

FIG. 11 shows another example of an image displayed on the display unit of the ophthalmic device according to the embodiment, where the image has not been subjected to offset processing.

FIG. 12 shows another example of an image displayed on the display unit of the ophthalmic device according to the embodiment, where a false fluorescein image is superimposed on an image captured while ring light is not projected.

FIG. 13A shows a graph for sum of deformations, where the graph and a threshold for determining a breakup time are not offset.

FIG. 13B shows a graph for sum of deformations, where a threshold for determining a breakup time is offset.

Embodiment

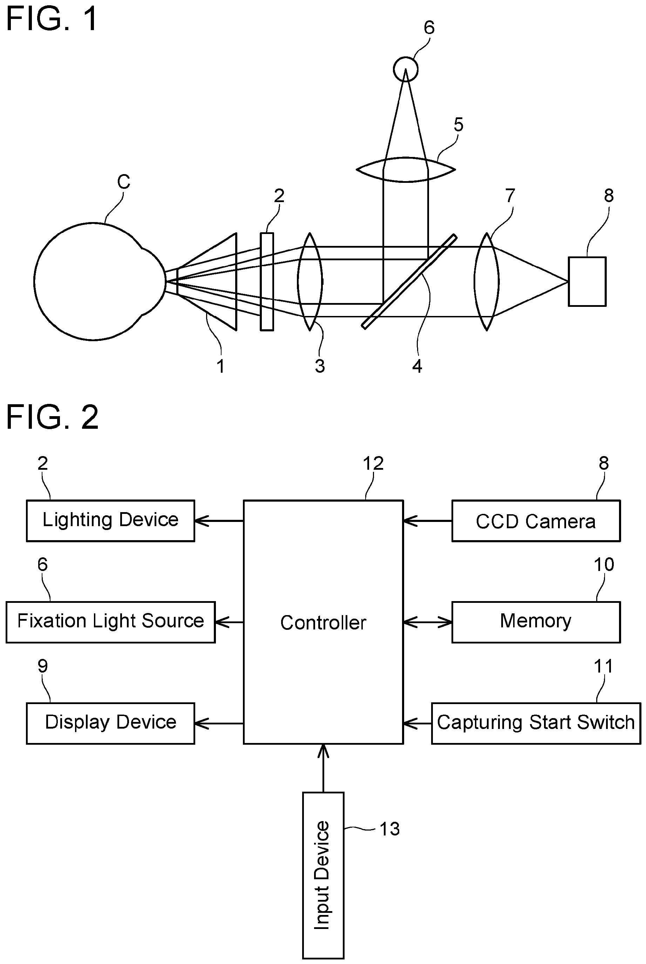

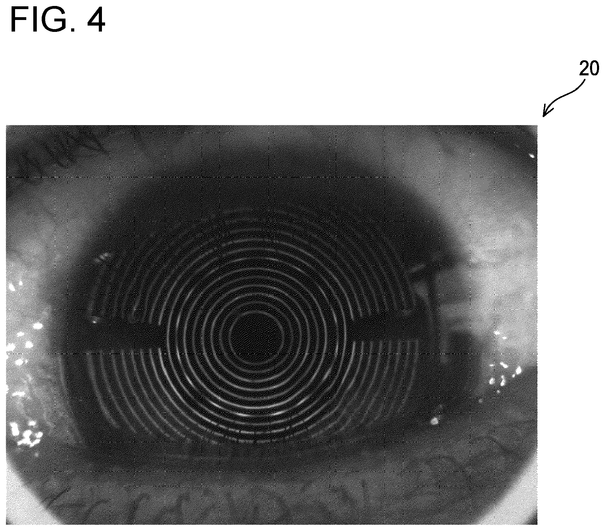

Hereinafter, an ophthalmic device (corneal shape analyzer) according to an embodiment is described. As shown in FIG. 1, the ophthalmic device according to the present embodiment comprises a cone 1 positioned to face the cornea of a subject eye C and a lighting device 2 (e.g., LED, etc.) disposed behind the cone 1. The cone 1 is a hollow and conic tubular body and is constituted of a transparent resin. A transparency film on which a pattern of concentric rings is printed is attached to an inner surface of the cone 1, while a light-reflective coating is applied to an outer surface of the cone 1. Therefore, light from the lighting device 2 disposed behind the cone 1 scatters inside the cone 1, is partially blocked by the transparency film attached to the inner surface, and then reaches the cornea of the subject eye C. Thus, pattern light having a pattern of concentric rings (ring light), such as the one shown in FIG. 4, is projected onto the cornea of the subject eye. As a method of projecting a desired pattern through the cone 1, the desired pattern may be directly engraved on the inner surface of the cone 1 and a light blocking coating may be applied to the engraved portions.

A lens 3 and a half mirror 4 are disposed behind the lighting device 2. Light from a fixation light source 6 enters the half mirror 4 through a lens 5. The light from the fixation light source 6 is reflected on the half mirror 4 and enters the cone 1 through the lens 3. This light passes through a center opening of the cone 1 and then enters the cornea of the subject eye C. The light from the fixation light source 6 is used as fixation light during cornea observation and is adjusted to be positioned at the center of the pattern of concentric rings formed by the cone 1. Reflected light from the corneal surface of the subject eye C (i.e., reflected image (reflected figure) of the pattern light having the pattern of concentric rings, which has been projected onto the corneal surface (ring images (ring figures)) and a reflected image (reflected figure) of the fixation light reflected) passes through the cone 1, the lens 3, and the half mirror 4, is subjected to focus adjustment in a lens 7, and is then observed by a CCD camera 8.

In order to accurately measure the corneal shape by the ophthalmic device described above, it is preferable to project the pattern of concentric rings about the apex of cornea of the subject eye C. For this purpose, during corneal shape measurement, the fixation light source 6 is turned on to make the subject fixate the light. A controller 12 (see FIG. 2) adjusts the position of the optical system relative to the subject eye C such that the light from the fixation light source 6 observed by the CCD camera 8 is positioned at the center of an image. In this way, the light having the pattern of concentric rings (ring light) can be projected about the apex of cornea of the subject eye C.

A control system of the ophthalmic device described above is described. As shown in FIG. 2, the ophthalmic device is controlled by the controller 12. The controller 12 can be configured of a computer comprising a CPU, ROM, RAM, etc. The CCD camera 8, a capturing start switch 11, an input device 13, and a memory 10 are connected to the controller 12. The capturing start switch 11 and the input device 13 are operated by an examiner. In response to examiner's operation on the capturing start switch 11, the controller 12 starts a predetermined program to measure chronological changes that occur in the tear film on the corneal surface over time. Data of images captured by the CCD camera 8 is input to the controller 12 and stored in the memory 10, etc. Further, the data of images captured by the CCD camera 8 is subjected to image processing by the controller 12. The controller 12 processes the data of images captured by the CCD camera 8 to provide useful information for dry eye diagnosis. The image processing by the controller 12 will be described in detail later.

The lighting device 2, the fixation light source 6, and a display device 9 are also connected to the controller 12. The controller 12 turns on/off the lighting device 2 and the fixation light source 6 and further displays images, etc. captured by the CCD camera 8 on the display device 9.

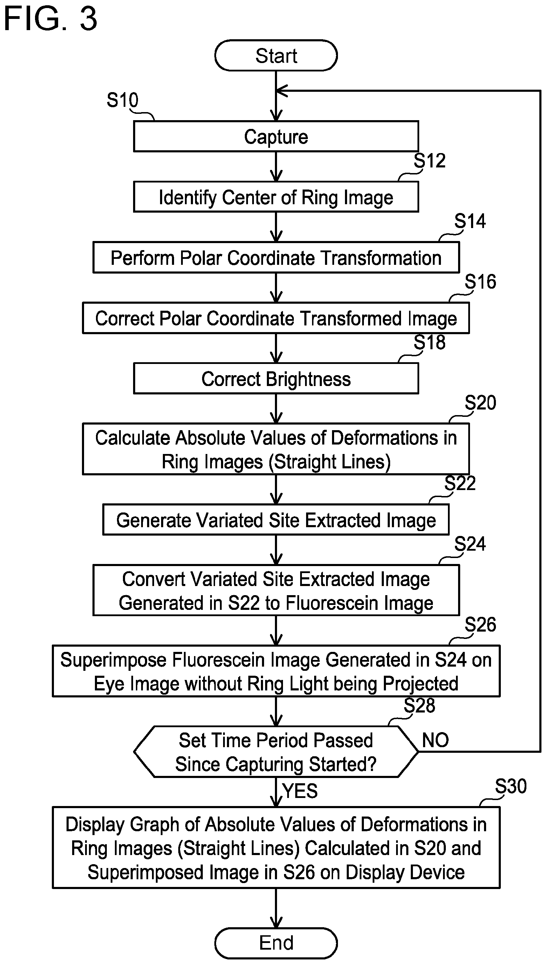

Next, steps for measuring a chronological change in the tear film on the corneal surface by using the above-described ophthalmic device are described. As shown in FIG. 3, first, images of an anterior part of the subject eye are captured by the ophthalmic device (S10). Specifically, the examiner turns on the fixation light source 6 and instructs the subject to fixate the fixation light. Once the fixation light source 6 is turned on, an image observed by the CCD camera 8 is displayed on the display device 9. The controller 12 adjusts the position of the optical system relative to the subject eye C such that the anterior part of the subject eye C displayed on the display device 9 is positioned at the center of the screen (i.e., such that the reflected light from the fixation light source 6 is positioned at the center of the cornea). After completing the positional adjustment of the optical system, the controller 12 turns on the lighting device 2 to project the ring light having the pattern of concentric rings onto the corneal surface of the subject eye C. Thus, the preparation for capturing images of the anterior part of the subject eye C is completed. Next, the examiner asks the subject to close his/her eyelids to make the tear film on the corneal surface even and then open the eyelids, while operating the capturing start switch 11. According to the examiner's operation on the capturing start switch 11, the controller 12 operates the CCD camera 8 to capture images of reflected image from the corneal surface.

Next, the controller 12 performs analytical processing on the images captured by the CCD camera 8 (S12 to S26). In the present embodiment, reflected images from the corneal surface are captured by 15 frames per second within a set time period (e.g., 10 seconds) from the start of operation on the capturing start switch 11. Then, steps S12 to S26 are performed on each of the captured images. FIG. 4 shows an example of an image captured by the CCD camera 8 (i.e., original image). The examiner asks the subject not to blink until the set time period elapses from the start of operation on the capturing start switch 11 (until the capturing is completed).

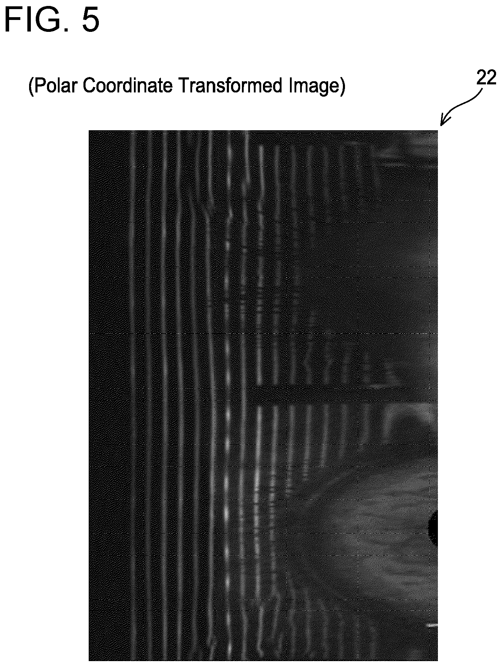

Hereinafter, steps S12 to S26 are described in detail. First, the controller 12 identifies the center of ring images in a captured image (S12) and performs polar coordinate transformation with the center as the origin (S14). In the present embodiment, the ring images are transformed over 240 pixels in the radial direction and by 1 degree in the circumferential direction to obtain a polar coordinate transformed image. As shown in FIG. 4, the captured image includes concentric ring images. Performing polar coordinate transformation on this image with the center of ring images as the origin results in the image shown in FIG. 5 (polar coordinate transformed image). As apparent from FIG. 5, in the polar coordinate transformed image, the ring images have been transformed to straight lines extending in up-down direction (in y-direction). This transformation from the ring images to the linearly extending images (which may be referred to as linear images below) facilitates step S16 and the subsequent steps. However, since the image is a reflected image from the corneal surface, the images may be curved line images that have long and short axes in directions represented by trigonometric function due to the severity of astigmatism, axis angle, or an error in the center identification. In step S12, the center of ring images may be identified by using, for example, the innermost ring image (i.e., the ring image with the smallest radius). Alternatively, the fixation light may be identified as the center if the fixation light is captured in the original image. The center of ring images identified in step S12 does not necessarily match the center for known corneal shape analysis (corneal topography).

Next, the controller 12 corrects the polar coordinate transformed image generated in step S14 (S16). In the captured image in step S10 (original image shown in FIG. 4), the image (ring images) may be distorted due to unstable tear film and/or the ring images may be partially missing due to shadow of eyelashes. Therefore, the linear images in the polar coordinate transformed image generated in step S14 may be also distorted and/or partially missing. For example, the image shown in FIG. 5 includes parts where the linear images are distorted due to unstable tear film and parts where the ring images are partially missing due to shadow of eyelashes. In step S 16, such artifacts in the polar coordinate transformed image, such as the distortions and partially missing parts of the linear images, are corrected (removed).

Specifically, for each pixel in the polar coordinate transformed image, an averaging region is set that is extended in a radial direction (an example of first direction) and a circumferential direction (an example of second direction) from the pixel and luminance of pixels in that range are averaged to correct the image. In the present embodiment, the ring light having the pattern of concentric rings is projected onto the corneal surface of the subject eye C. Thus, if the ring images are not distorted or partially missing, the linear images in the polar coordinate transformed image should be substantially straight line images extending in the up-down direction (in y-direction). Therefore, for example, when a certain reference pixel is compared with another pixel whose circumferential position is different from that of the reference pixel, the distances between the rings do not change much near the position of the reference pixel. That is, the ring images do not change much in their radial positions. Further, even when the subject eye is astigmatic, the distances between the rings do not change much in an angle direction. In view of this, by taking advantage of the characteristic that the ring images do not change much in their radial positions when they are not distorted nor partially missing, distorted and/or missing parts of the ring images are corrected and complemented.

A variety of methods can be used for the calculation of luminance of each pixel in step S16. For example, a linear region that is circumferentially extended by a predetermined extent from a pixel for which the luminance is to be calculated may be set as an averaging region, and luminance of pixels within the averaging region may be averaged. Alternatively, a rectangular region that is radially and circumferentially extended simply by predetermined extents from a pixel for which the luminance is to be calculated may be set as an averaging region, and luminance of pixels within the averaging region may be averaged.

Alternatively, average values may be calculated in regions radially near a pixel for which the luminance is to be calculated to identify an averaging region with the smallest error, and luminance of pixels within the identified averaging region may be averaged. An example of this method is specifically described using formulas 1 and 2, where the circumferential position of a pixel for which the luminance is to be calculated is a, the radial position of the pixel is r, and the luminance of the pixel is (a, r). The average value calculation range is $\pm r_w$ in the radial direction and $\pm a_w$ in the circumferential direction. As described above, if the ring images are free of distortions and missing parts, the ring images hardly change in their radial positions, whereas if the ring images are distorted and/or partially missing, the ring images are radially displaced. The radial displacement of ring images is varied depending on the circumferential position (angle a) and radial position r. Therefore, this displacement of ring images is represented as h (a, r). As described, the displacement h (a, r) is a function whose value varies depending on the angle a and the radius r. In order to identify an averaging region with the smallest error, an angle a' and displacement h (a', r) that minimize S (a', r) shown in Formula 1 are calculated. Here, the angle a' is an angle near the angle a.

Formula 1

a: angle [°]
r: radius [pixel]
$\pm r_w$: calculation range in radial direction [pixel]
$\pm a_w$: calculation range in angular direction [°]
h (a, r): displacement [pixel]

The formula below is calculated for target pixel P (a, r). a' is an angle near angle a.

$$S(a',r) = \sum_{r'=-r_w}^{r_w} |P(a', r+r'+h(a',r)) - P(a, r+r')|$$

After the angle a' and the displacement h (a', r) are calculated, an averaged luminance P' (a, r) of pixel (a, r) is calculated according to the formula 2. By averaging luminance of each pixel in the polar coordinate transformed image as above, distortions and partial missing of the ring images are corrected.

Formula 2

$$P'(a,r) = \frac{\left(\sum_{a'=a-a_w}^{a+a_w} P(a', r + h(a', r))\right)}{2 \times a_w + 1}$$

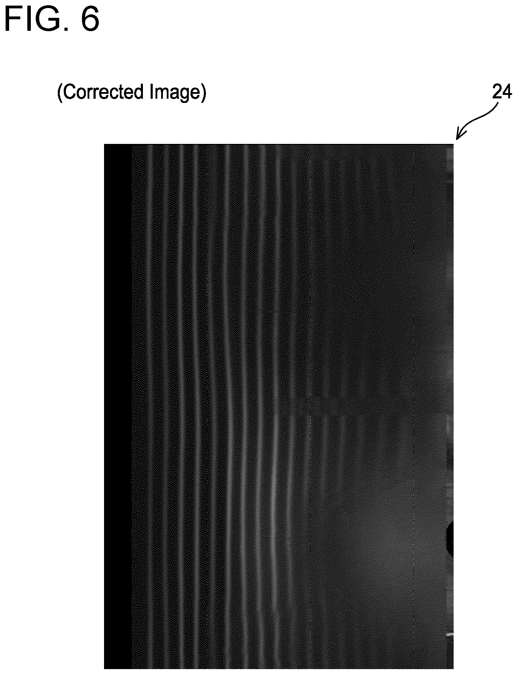

FIG. 6 shows a corrected image corrected according to the formulas 1 and 2. As apparent from the comparison between FIGS. 5 and 6, in the corrected image, the distortions of the linear images have been corrected and the missing parts of the linear images due to shadow of eyelashes have been complemented. However, even in the corrected image, the linear images remain distorted in a lower part of the image. This is presumably because the linear images in the image prior to the correction (in the image shown in FIG. 5) are significantly distorted, but the liner images have been corrected enough not to cause adverse effects in practical use. In the corrected image shown in FIG6, missing portions of the rings positioned at angle positions of 0 degrees and 180 degrees are complemented with a little deviation. This is because there is no data for regions external to the start positions of the missing portions. Further, there is no need to restore regions for which data is lacking due to upper and lower eyelids, and thus these regions remain unrestored.

Next, the controller 12 performs steps for outputting useful information for diagnosis of tear film condition based on the corrected image corrected in step S16 (steps S18 to S26). For steps S18 to S26, regions of the captured image and the corrected image of step S16 that are not interrupted by the upper and lower eyelids are cut out, and steps S18 to S26 are performed on these cut-out regions.

First, the controller 12 corrects the luminance of the corrected image of step S16 and also corrects the luminance of the polar coordinate transformed image prior to the correction of step S16 (S18). In the present embodiment, in order to detect a luminance decrease and displacement of the ring images due to unstable tear film, the corrected image of step S16 is compared with the polar coordinate transformed image prior to the correction of step S16 (which may be referred to as the pre-correction image). The luminance of each image is corrected to appropriately compare the two images.

Specifically, the controller 12 first obtains a luminance waveform, for the corrected image of step S16, that shows a relationship between "radial position" and "luminance" for each angle, and then calculates a comprehensive straight line that includes the maximum and minimum values of the luminance waveform. Here, assuming that the comprehensive straight line calculated from the corrected image includes upper and lower limits of the luminance of the pre-correction image, a comprehensive straight line for the pre-correction image is created from the corrected image. That is, if the upper and lower limits of the pre-correction image exceed the comprehensive straight line of the corrected image, the upper and lower limits of the pre-correction image are used to correct the comprehensive straight line of the corrected image and create the comprehensive straight line for the pre-correction image. In the corrected image of step S16, the positions of the ring images have been corrected to their right positions. If the ring images are free from distortions due to unstable tear film, the positions of ring images in the pre-correction image and the positions of ring images in the corrected image should match each other and thus should not be offset significantly. Therefore, a peak position of the ring images in the pre-correction image is detected near a peak position of the corrected image.

After calculating the comprehensive straight lines as above, the controller 12 expands the luminance waveforms to a predetermined luminance range (e.g., gray levels 20 to 200 (full span: gray levels 0 to 255)) by using the created comprehensive straight lines. Preferably, the luminance waveforms are expanded to a range narrower than the full span. The expansion of the luminance waveforms to a range narrower than the full span prevents the luminance from exceeding the upper and lower limits in the course of calculation. The luminance waveform of the corrected image and a luminance waveform of the pre-correction image are expanded such that luminance peak heights of the ring images are aligned. The luminance correction in step S18 is described in detail in Japanese Patent Application Publication No. 2020-10986.

Next, the controller 12 calculates a sum of absolute values of deformations in the ring images (linear images) (which may be referred to as "sum of deformations" hereinafter) by subtracting the luminance-corrected corrected image from the luminance-corrected pre-correction image (S20). Specifically, the controller 12 first calculates, for each pixel in the pre-correction image, a luminance difference between the pixel in the pre-correction image and a pixel in the corrected image corresponding to that pixel. For example, in a case the ring images are not distorted nor partially missing, when a pixel in the pre-correction image is bright or dark (i.e., when the pixel is on or off the linear image), its corresponding pixel in the corrected image is accordingly bright or dark (i.e., the pixel is accordingly on or off the linear image). Therefore, a luminance difference between these corresponding pixels in the pre-correction image and the corrected image is small. On the other hand, in a case the ring images are distorted and/or partially missing, when a pixel in the pre-correction image is bright or dark (i.e., when the pixel is on or off the linear image), its corresponding pixel in the corrected image is dark or bright (i.e., the pixel is off or on the linear image). Therefore, a luminance difference between these corresponding pixels in the pre-correction image and the corrected image is large. Further, in a case the ring images are distorted and/or missing to a larger extent, more pixels in the pre-correction image have large luminance differences from their corresponding pixels in the corrected image. In the present embodiment, for each pixel in the pre-correction image, a luminance difference between the pixel in the pre-correction image and its corresponding pixel in the corrected image is calculated and absolute values of the calculated luminance differences are summed to calculate the "sum of deformations".

In the case the ring images are distorted and/or partially missing, when a pixel in the pre-correction image is bright (i.e., the pixel is on a ring figured in the pre-correction image), its corresponding pixel in the corrected image is dark (i.e., since the ring images are distorted, the corresponding pixel is offset from a ring image and thus is dark in the corrected image). Since the ring images are radially arranged at predetermined intervals, a certain pixel in the pre-correction image is bright and a pixel located radially apart from the certain pixel by a half of the predetermined interval is dark. Therefore, a combination of a bright pixel in the pre-correction image and its corresponding dark pixel in the corrected image is paired with a combination of a dark pixel in the pre-correction image and its corresponding bright pixel in the corrected image. Thus, only one of these combinations may be counted for evaluation on the sum of deformations. In the present embodiment, only the combination of a bright pixel in the pre-correction image and its corresponding dark pixel in the corrected image is counted for calculation of the sum of deformations. Thus, missing parts of the ring images due to eyelashes (i.e., dark pixel in the pre-correction image and bright pixel in the corrected image) are not counted for calculation of the sum of deformations. Therefore, only deformations of the ring images (linear images) are appropriately evaluated.

The method for calculating "sum of deformations" is not limited to the above method but a variety of methods can be used. For example, a ring image (bright part) in the pre-correction image is narrowed into a line, a ring image (bright part) in the corrected image is narrowed into a line, and displacement and a luminance difference between these two lines are quantified. Then, a predetermined weighting is applied to each of the displacement of the two lines (absolute value) and the illuminance difference of the two lines (absolute value), and these are added up to calculate the sum of deformations.

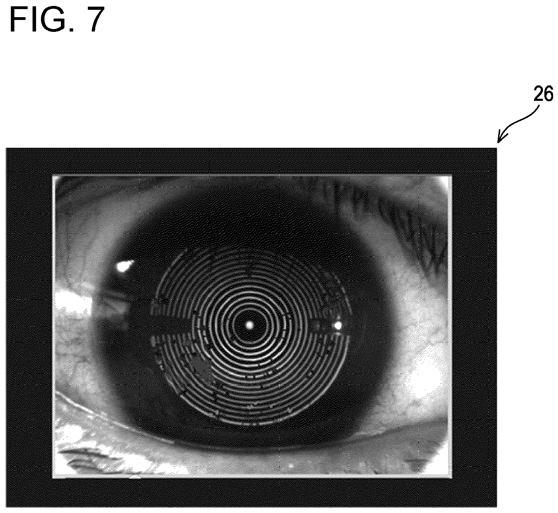

Next, the controller 12 generates a variated site extracted image in which a variated site is extracted (S22) by subtracting the luminance-corrected corrected image from the luminance-corrected pre-correction image as described above. That is, with no change in the state of tear film, the luminance and positions of ring images in the luminance-corrected pre-correction image match the luminance and positions of ring images in the luminance-corrected corrected image. Contrary to this, with unstable tear film, the luminance and positions of ring images change, resulting in variated sites between the two images. In step S22, changed portions of the tear film (variated sites) are extracted from the difference between the two images. A difference image showing the difference between the two images is a polar coordinate transformed image, and it is difficult to understand, from that image, where on the corneal surface the changes occurred. Therefore, the difference image showing the difference between the two images may be transformed to Cartesian coordinates and the variated sites may be plotted on an eye image. FIG. 7 shows an eye image with the variated sites plotted thereon. As shown in FIG. 7, the eye image with the variated sites plotted thereon allows for easier understanding on where on the eye the tear film is unstable. Although the ring images remain in the image shown in FIG. 7, the image may not include the ring images. That is, the ring images may be removed from a captured image, and the extracted variated sites may be superimposed on the image from which the ring images have been removed. Alternatively, an image of the subject eye C may be captured while the ring light is not projected thereon and the extracted variated sites may be superimposed on that image. Removing the ring images allows the examiner to more easily distinguish boundaries (edges) of the iris and pupil and thus to know where on the corneal surface the variated sites are present.

Next, the controller 12 converts the variated site extracted image generated in step S22 to a false fluorescein image in which magnitudes of the changes are represented in luminance (S24). In step S22, the variated site extracted image is generated on which the variated sites of the tear film (i.e., variated sites between the pre-correction image and the corrected image) are shown. In the variated site extracted image, magnitudes of changes in the tear film are larger for where the luminance differences between the pre-correction image and the corrected image are larger, while magnitudes of changes in the tear film are smaller for where the luminance differences between the pre-correction image and the corrected image are smaller. Here, the fluorescein image is generally used in dry eye examinations, in which luminance of stained portions decrease according to the degrees of breakup of the tear film. In the present embodiment, the variated site extracted image is converted to the false fluorescein image by lowering the luminance for portions having larger magnitudes of changes. That is, in step S24, the variated site extracted image is converted to the false fluorescein image by giving different shades depending on the magnitude of changes in color tones similar to those of fluorescein.

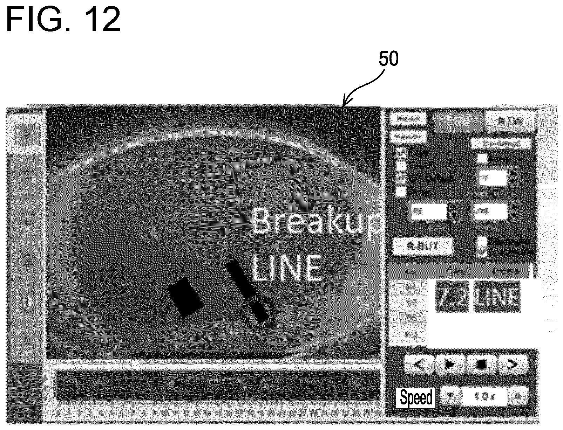

Next, the controller 12 superimposes the false fluorescein image generated in step S24 onto an eye image that does not include ring images (S26). The false fluorescein image generated in step S24 is a polar coordinate transformed image, and it is difficult to understand, from that image, where on the corneal surface the changes occurred. Therefore, the false fluorescein image generated in step S24 is transformed to Cartesian coordinates and is then superimposed onto the eye image. The resulting image is similar to a fluorescein image obtained in dry eye examination and allows the examiner to know where on the corneal surface the variated sites are present more easily. Thus, the useful image for dry eye diagnosis can be provided. The eye image onto which the false fluorescein image is superimposed may be a captured image from which the ring images have been removed or an image of the subject eye C captured while the ring light is not projected thereon. FIG. 12 shows an exemplary image generated in step S26. As shown in FIG. 12, since the image in which the luminance of variated sites has been differentiated in color tones similar to those of fluorescein is superimposed on the eye image, this image looks similar to an image obtained in dry eye examination and allows the examiner to easily know where on the eye the tear film is unstable. The generation of the false fluorescein image in steps S24 and S26 is described in Japanese Patent Application Publication No. 2020-10986 in detail.

Next, the controller 12 determines whether the set time period (e.g., 10 seconds) has elapsed since the start of operation on the capturing start switch 11 (S28). If 10 seconds has elapsed since the start of image capturing (YES in S28), the controller 12 proceeds to step S30. If 10 seconds have not elapsed yet since the start of image capturing (NO in S28), the controller 12 returns to step S10 and repeats the steps from step S10. Thus, from the start of image capturing until the set time period has elapsed, reflected images from the corneal surface are captured at predetermined time intervals by the CCD camera 8, and for each of the captured images, a "sum of deformations" is calculated and a false fluorescein image is generated.

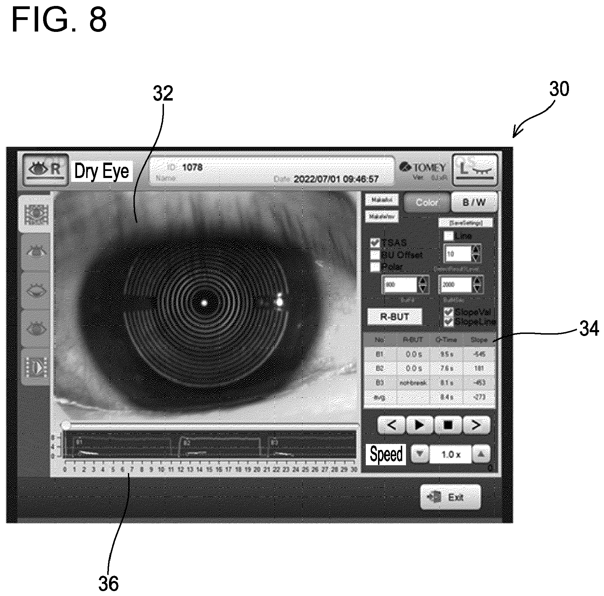

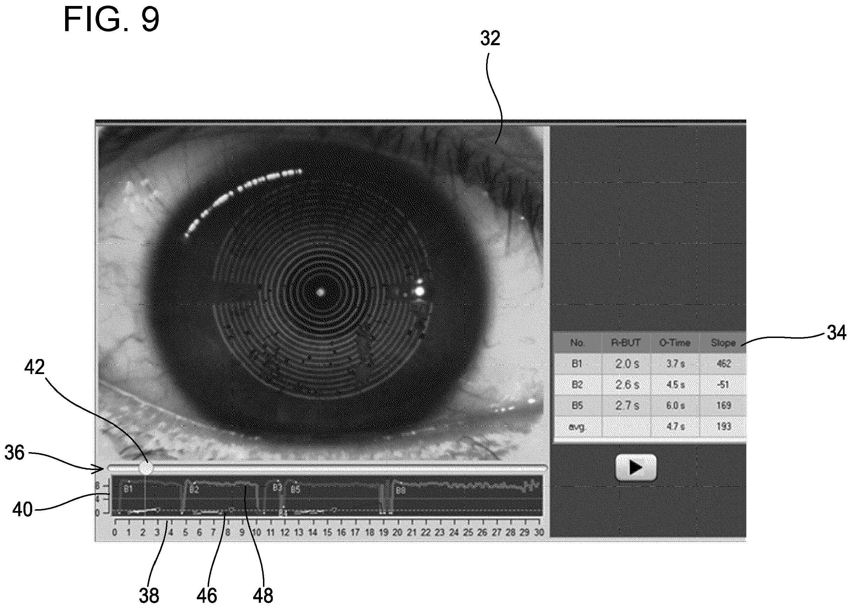

If determining YES in step S28, the controller 12 displays a graph showing sums of deformations calculated for the respective captured images in step S20 and the various images generated in step S22 to step S26 on the display device 9 (S30). Since the graph showing the sums of deformations and the eye image on which the variated sites are superimposed are displayed on a single screen, the useful information for dry eye diagnosis can be provided. There are various types of dry eye, such as the aqueous deficient type dry eye, the decreased wettability type dry eye, and the increased evaporation type dry eye, and therapeutic strategy varies depending on the type. In order to determine the dry eye type, it is necessary to not only measure a time until the tear film breaks (so-called breakup time) but also observe a breakup pattern of the tear film (how the tear film breaks up). For example, in the aqueous deficient type dry eye, the tear film on the corneal surface breaks entirely, while in the decreased wettability type dry eye, the tear film on the corneal surface breaks partially. Therefore, the breakup pattern of tear film needs to be observed. Further, in dry eye, the sum of deformations increases over time since the tear film usually breaks over time after the eyelids open. However, in the decreased wettability type dry eye, unevenness of the tear film is greatest immediately after the eyelids open and the unevenness of the tear film decreases over time. Therefore, it is also necessary to see how unevenness of the tear film changes over time. In view of this, as shown in FIGS. 8 and 9, in the ophthalmic device according to the present embodiment, the display device 9 displays the graph showing the sums of deformations and the eye image on which the variated sites are superimposed.

As shown in FIGS. 8 and 9, an eye image 32 on which the variated sites are superimposed, a table 34 showing breakup times, and a graph 36 showing a chronological change of the sums of deformations are displayed on a screen 30 of the display device 9. The eye image 32 and the graph 36 are arranged one above the other, and the table 34 is arranged next to the eye image 32. Further, a seek bar 42 is displayed between the eye image 32 and the graph 36. The examiner operates the input device 13 to horizontally move a slider of the seek bar 42, so that an eye image 32 that was captured at the time specified by the slider is displayed on the display device 9. A line extending downward from the slider is displayed in the graph 36 as a vertical line indicating a time.

Various images of eye 32 can be displayed on the display device 9. The examiner operates the input device 13 to display desired image(s) of eye 32 on the display device 9. For example, as shown in FIG. 8, the eye image captured in step S10 (i.e., original image) can be displayed, or as shown in FIG. 9, the eye image captured in step S10 on which the variated sites are superimposed can be displayed, or as shown in FIG. 12, the image generated in step S26 (i.e., the eye image from which the ring images have been removed and on which the false fluorescein image is superimposed) can be displayed. As described above, the capturing time at which an eye image 32 displayed on the display device 9 was captured can be switched by horizontally moving the slider of the seek bar 42. This allows the examiner to observe how the variated sites of the tear film (i.e., dark regions with decreased luminance) changed. The examiner is easily able to know, for example, how adjacent variated sites (dark regions) of the tear film joined, how a single variated site (dark region) emerged, and how variated sites (dark regions) of the tear film expand and how fast they expand, and thus can determine the type of dry eye. When the area of a variated site (dark region) of the tear film reaches or exceeds an area preset therefor in the eye image 32, a mark indicating that the area of the variated site is equal to or larger than the preset area may be superimposed on that variated site. This allows the examiner to easily grasp the size (area) of the variated site. Alternatively, since areas of respective variated sites (dark regions) of the tear film can be calculated, a plurality of sections may be preset for the subject eye and a total area of variated sites (dark regions) in each of the sections may be calculated and displayed.

On the display device 9, an image observed by a slit-lamp microscope on which only the variated sites (low-luminance parts) of the false fluorescein image generated in step S24 are superimposed may be displayed. This allows a similar image to an image of fluorescein stained eye observed by a slit-lamp microscope to be displayed on the display device 9. This facilitates dry eye diagnosis for the doctor. In order to display the above-mentioned image on the display device 9, the ophthalmic device needs to obtain an image observed by a slit-lamp microscope. For this purpose, for example, the ophthalmic device may include an image capturing device for capturing an image observed by a slit-lamp microscope or an image observed by a slit-lamp microscope may be input to the controller 12 from an external device. In the above example, an image observed by a slit-lamp microscope on which only variated sites (low-luminance parts) of the false fluorescein image are superimposed is displayed, however, other aspects are conceivable. For example, the false fluorescein image may be superimposed on an image observed by a slit-lamp microscope, and one of the image observed by a slit-lamp microscope and the false fluorescein image may be switchably displayed by operating the input device 13.

The table 34 shows breakup times measured in each measurement shown in the graph 36 showing a chronological change of the sums of deformations. For example, in the screen 30 shown in FIG. 8, the graph 36 shows three measurements B1, B2, and B3. Accordingly, the table 34 shows breakup times (abbreviated as "BUT" in the table 34) for the measurements B1, B2, and B3, respectively. In FIG. 8, the breakup times for the measurements B1, B2, and B3 are "0" (zero), which means that there was no breakup in these measurements. Whether there was a breakup or not may be determined based on the sum of deformations calculated in step S20. That is, it may be determined that there was a breakup when the sum of deformations calculated in step S20 is equal to or greater than a predetermined threshold. By this determination method, whether there was a breakup or not can be objectively determined using the sum of deformations. However, the method of determining whether there was a breakup or not is not limited to the above method, and various other methods may be used instead. For example, it may be determined that there was a breakup when the area of variated sites (low-illuminance parts) of the false fluorescein image reaches or exceeds a predetermined area. In the table 34, "O-Time" means eyelids opening time. For example, in the measurement B1 shown in FIG. 9, the eyelids opening time is 3.7 seconds and the breakup time is 2.0 seconds. This means that in the measurement B1 shown in FIG. 9, the subject could keep his/her eyelids open for 1.7 seconds after the end of breakup time.

The table 34 also shows, for each measurement, a slope of the sum of deformations (Slope in the table 34) over a predetermined time period (e.g., 3 seconds (however, the examiner can set any time period)) from the start of eyelids opening. That is, the table 34 shows, for each measurement, a value (i.e., slope) that is calculated by dividing the difference between the sum of deformations at the start of eyelids opening and the sum of deformations at the time when the predetermined time period has elapsed from the start of eyelids opening by the predetermined time period. This allows the examiner to grasp whether the sum of deformations increased or decreased over the predetermined time period from the start of eyelids opening. As explained above, in case of decreased wettability type dry eye, the sum of deformations has a large value immediately after the start of eyelids opening and it decreases over time. Thus, the slopes of the sums of deformations shown in the table 34 facilitate a determination on whether the subject eye is a decreased wettability type dry eye or not.

As shown in FIG. 9, the graph 36 shows a chronological change of the sums of deformations, where a vertical axis 40 represents "sum of deformations" and a horizontal axis 38 represents "time". A distance 48 between the eyelids (referred to as "eyelids distance 48" hereinafter) of the subject eye is superimposed on the graph 36. The eyelids distance 48 is "0" while the eyelids of subject eye are closed, whereas it constantly hovers at a predetermined value while the eyelids are open. The eyelids distance of the subject eye can be calculated, for example, from the images of eye captured in step S10. Upper and lower parts of the eye are detected based on the luminance of images of eye captured in step S10 and the distance between these parts is calculated as an eyelids distance. While the calculated eyelids distance is "0", it is determined that the eyelids are closed, and the end of time period during which the eyelids distance is zero is identified as "eyelids opening start point". Further, the point of time at which the eyelids distance becomes maximum after the eyelids opening start point is identified as "eyelids opening end point". As described above, the controller 12 functions as "eyelids opening detector" that detects opening of the eyelids of the subject eye by the controller 12 performing image processing on the images of eye captured in step S 10. In the embodiment described above, the image processing (S12 to S26) is performed on all the images captured in step S10, however, the present technology is not limited thereto. For example, the image processing of S12 to S26 may be performed only on images captured after a predetermined time period (e.g., 0.5 seconds) from detection of the eyelids opening end point. This narrows the target of the image processing to the images captured after the eyelids are opened, and thus the image processing is performed efficiently.

As described above, since the graph 36 shows how the sums of deformations chronologically change, it facilitates understanding on how the sums of deformations change (e.g., increase or decrease) over a predetermined time period immediately after opening of the eyelids and thus facilitates a determination on whether the subject eye is a decreased wettability type dry eye or not. The graph 36 may be partially displayed in an enlarged manner. Such enlarged display of the graph 36 facilitates understanding on how the sums of deformations change.

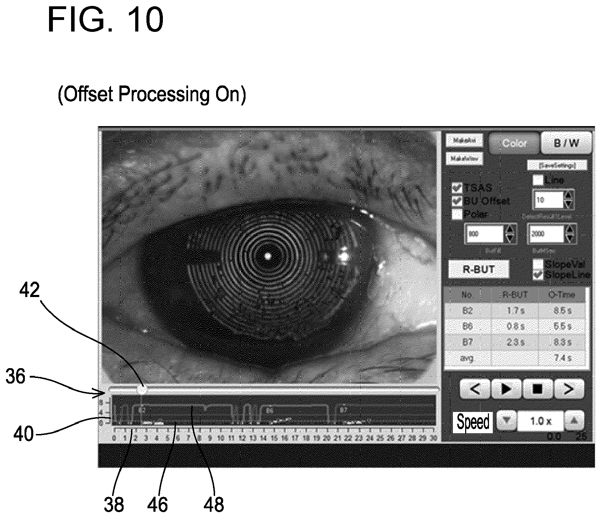

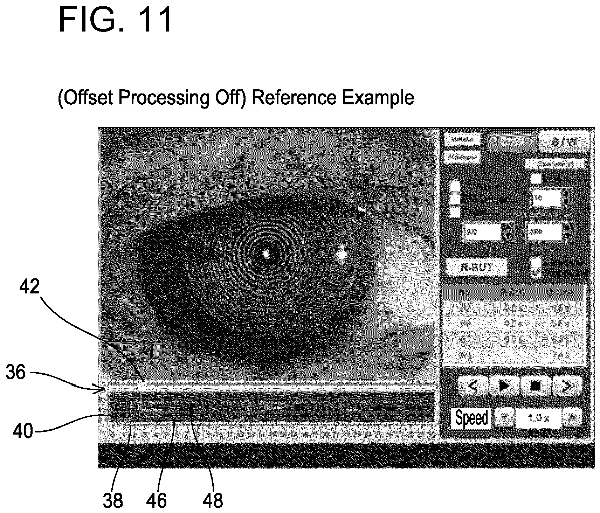

As mentioned above, a certain subject eye has a significantly uneven tear film immediately after the eyelids open, for example, due to its scratched corneal surface, etc., and thus the sum of deformations for an image captured immediately after the eyelids open is large. For such an eye, the sum of deformations for an image captured immediately after the eyelids open should be used as a reference in order to see how the sums of deformations change immediately after the eyelids open. For this reason, for example, an offset is calculated that makes the sum of deformations for the image captured immediately after the eyelids open to becomes "zero", and each captured image is offset by the calculated offset. This offset processing allows for easy understanding on how the sums of deformations change immediately after the eyelids open. FIG. 10 shows a graph 36 for the sums of deformations to which the offset processing has been performed, while FIG. 11 shows a graph 36 for the sums of deformations to which the offset processing has not been performed. In the graph 36 to which the offset processing has been performed shown in FIG. 10, the sum of deformations immediately after the eyelids open is "0" and the following sums of deformations change. On the other hand, in the graph 36 to which the offset processing has not been performed shown in FIG. 11, the sum of deformations immediately after the eyelids open has a large value and it is difficult to see the following trend (i.e., an increasing trend or a decreasing trend). As apparent from FIGS. 10 and 11, the offset processing allows the examiner to easily see the trend of sums of deformations and accurately determine the type of dry eye the subject eye has.

In a decreased wettability type dry eye, the tear film is significantly uneven immediately after the eyelids open and the unevenness of the tear film decreases over time. In case where a breakup time is to be determined based on whether the sum of deformations reaches a threshold or not, subjecting the sum of deformations to the offset processing makes the breakup time determination impossible. Therefore, whether the sums of deformations for images captured immediately after the eyelids open show an increasing trend or a decreasing trend is determined, and if they show a decreasing trend, the subject eye is diagnosed as decreased wettability type dry eye and the offset processing is not performed. In this way, the breakup time determination is possible for the decreased wettability type dry eye.

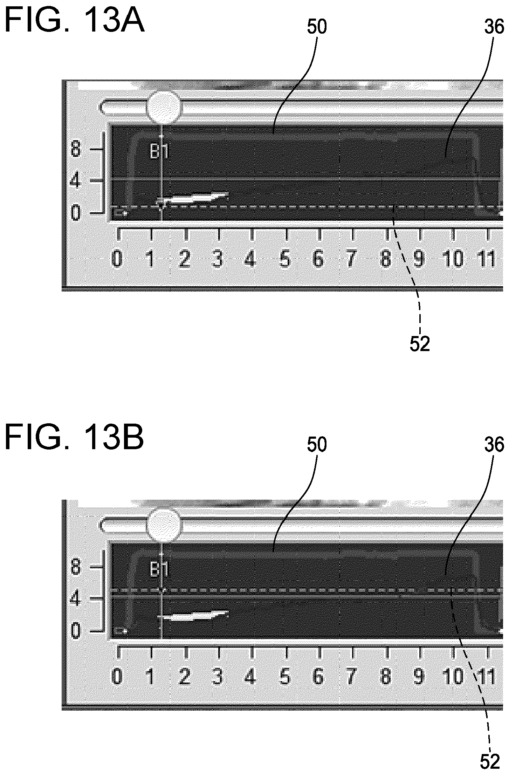

As shown in FIGS.13A and 13B, a threshold for determining a breakup time may be offset to above instead of offsetting the graph 36 for the sums of deformations. FIG. 13A shows the graph 36 for the sums of deformations, a graph 50 for the eyelids distance, and a threshold 52, and FIG. 13B shows the threshold 52 that is offset to above compared to its position shown in FIG. 13A. In this case as well, the examiner can easily see how the sums of deformations shift after the eyelids open from the graph 36 for the sums of deformations and thus can properly determine the type of dry eye.

Superficial punctate keratopathy can be found through an eye image captured while the ring light is not projected. In the ophthalmic device according to the present embodiment, the display device 9 can display an eye image captured while the ring light is not projected. Therefore, the ophthalmic device according to the present embodiment can provide useful information for determining whether the eye is suffering from superficial punctate keratopathy or not. Further, the ophthalmic device according to the present embodiment can provide information for determining whether the eye is a dry eye or not based on breakup time. Further, as a conventional diagnostic approach for dry eye, there is an approach to determine the type of dry eye based on the breakup pattern of cornea staining and pick a therapeutic strategy. The ophthalmic device according to the present embodiment can provide information for determining the type of dry eye based on how variated sites (low-luminance dark regions) of the tear film changes (i.e., breakup pattern), which is displayed in the display device 9. Further, the ophthalmic device according to the present embodiment can provide information for determining whether the eye is a decreased wettability type dry eye or not based on a slope of sums of deformations. As described above, the ophthalmic device according to the present embodiment can provide these information required to diagnose a dry eye through only a single examination. In case where an eye is checked following the conventional dry eye practice guidelines, the subject eye has to be subjected to multiple examinations and whether the subject eye is a dry eye or not has to be determined comprehensively based on the examination results. The ophthalmic device according to the present embodiment can obtain results of such multiple examinations by performing only a single examination, and thus can efficiently obtain examination results required to diagnose a dry eye. Consequently, whether the eye is a dry eye or not can be accurately determined and even a therapeutic strategy can be specified through a single examination.

While specific examples of the present disclosure have been described above in detail, these examples are merely illustrative and place no limitation on the scope of the patent claims. The technology described in the patent claims also encompasses various changes and modifications to the specific examples described above.

In the embodiment described above, images of the subject eye are captured at predetermined time intervals while the ring light is projected thereon, however, the technology disclosed herein is not limited thereto. For example, images of the subject eye may be captured while the ring light is turned on and off alternatively.

In the embodiment described above, the ring light having a pattern of concentric rings is projected onto the corneal surface of the subject eye, however, the pattern light projected onto the corneal surface of the subject eye is not limited to the ring light. Pattern light having any pattern of, for example, vertical lines, horizontal lines, grids, etc. separated from each other at certain intervals to cover the corneal surface may be used. Even with such pattern light, an appropriate averaging region can be easily set for the correction of an image. Therefore, the same analysis as the one described in connection with the embodiment above can be performed.

In the embodiment described above, polar coordinate transformation is performed on each captured image and the following analysis is performed using the polar coordinate transformed images, however, the technology disclosed herein is not limited thereto. For example, each captured image itself may be analyzed to detect displacement of ring images, etc.

The technical elements explained in the present description or drawings provide technical utility either independently or through various combinations. The present disclosure is not limited to the combinations described at the time the claims are filed. Further, the purpose of the examples illustrated by the present description or drawings is to satisfy multiple objectives simultaneously, and satisfying any one of those objectives gives technical utility to the present disclosure.

**Claims**

1. An ophthalmic device comprising:

   a projection unit (1, 2) configured to project pattern light onto a corneal surface of a subject eye (C);
   a first image capturing unit (8) configured to capture a reflected image of the pattern light reflected from the corneal surface;
   a controller (12) configured to cause the first image capturing unit (8) to repeatedly capture a reflected image of the pattern light reflected from the corneal surface at predetermined time intervals; and
   an image processing unit (12) configured to process captured images captured by the first image capturing unit (8),
   wherein
   the pattern light is light having a preset pattern formed of lines, and
   the image processing unit (12) is further configured to calculate, for each of the captured images, a sum of absolute values of deformations in lines of a pattern image created by the pattern light in the captured image.

2. The ophthalmic device according to claim 1, further comprising an output unit (9) configured to chronologically output the sums of the absolute values of deformations from a time when capturing of the images is started or a time after a predetermined time period from the start of the capturing of the images.

3. The ophthalmic device according to claim 2, wherein

   the output unit (9) is further configured to output a chronological change in the sums of the absolute values of deformations over time, and
   the chronological change is a change in the sums of the absolute values of deformations over a predetermined time period from the time when the capturing of the images is started or the time after a predetermined time period from the start of the capturing of the images.

4. The ophthalmic device according to claim 2 or 3, further comprising:

   a second image capturing unit (8) configured to capture an image of the subject eye (C) while the pattern light is not projected; and
   a display unit (9) configured to display the image of the subject eye (C),
   wherein the display unit (9) is further configured to display a graph (36) showing the chronological change in the sums of the absolute values of deformations together with the image of the subject eye.

5. The ophthalmic device according to claim 4, wherein

   the image processing unit (12) is further configured to identify, in each of the captured images, a variated site where the sum of the absolute values of deformations exceeds a predetermined value, and
   the display unit (9) is further configured to display the variated site such that the variated site is superimposed on the image of the subject eye.

6. The ophthalmic device according to claim 5, wherein
   the display unit (9) is further configured to display a mark indicating a time when a captured image including the variated site was captured such that the mark is superimposed on the graph (36) showing the chronological change in the sums of the absolute values of deformations.

7. The ophthalmic device according to any one of claims 1 to 6, further comprising an eyelids detection unit (12) configured to detect opening of eyelids of the subject eye (C),

wherein the image processing unit (12) is further configured to correct the sums of the absolute values of deformations calculated for the captured images such that the sum of the absolute values of deformations calculated for a captured image captured immediately after the eyelids detection unit (12) detected opening of the eyelids of the subject eye (C) becomes a predetermined value.

# FIG. 1

# FIG. 2

# FIG. 3

```
                    ┌──────────────┐
                    │    Start     │
                    └──────┬───────┘
                           │ ◄────────────────┐
 S10                       ▼                  │
        ┌──────────────────────────────────┐ │
        │            Capture               │ │
        └──────────────────┬───────────────┘ │
                           │        S12       │
        ┌──────────────────▼───────────────┐ │
        │     Identify Center of Ring Image │ │
        └──────────────────┬───────────────┘ │
                           │        S14       │
        ┌──────────────────▼───────────────┐ │
        │  Perform Polar Coordinate Transformation │
        └──────────────────┬───────────────┘ │
                           │        S16       │
        ┌──────────────────▼───────────────┐ │
        │ Correct Polar Coordinate Transformed Image │
        └──────────────────┬───────────────┘ │
                           │        S18       │
        ┌──────────────────▼───────────────┐ │
        │        Correct Brightness        │ │
        └──────────────────┬───────────────┘ │
                           │        S20       │
        ┌──────────────────▼───────────────┐ │
        │ Calculate Absolute Values of Deformations in │
        │   Ring Images (Straight Lines)   │ │
        └──────────────────┬───────────────┘ │
                           │        S22       │
        ┌──────────────────▼───────────────┐ │
        │ Generate Variated Site Extracted Image │
        └──────────────────┬───────────────┘ │
                           │        S24       │
        ┌──────────────────▼───────────────┐ │
        │  Convert Variated Site Extracted Image │
        │ Generated in S22 to Fluorescein Image │
        └──────────────────┬───────────────┘ │
                           │        S26       │
        ┌──────────────────▼───────────────┐ │
        │ Superimpose Fluorescein Image Generated in S24 on │
        │ Eye Image without Ring Light being Projected │
        └──────────────────┬───────────────┘ │
                           │        S28   NO  │
                      ◄────▼────►────────────►┘
                      Set Time Period Passed
                      Since Capturing Started?
                           │ YES
                           │        S30
        ┌──────────────────▼───────────────┐
        │ Display Graph of Absolute Values of Deformations in │
        │ Ring Images (Straight Lines) Calculated in S20 and │
        │ Superimposed Image in S26 on Display Device │
        └──────────────────┬───────────────┘
                           ▼
                    ┌──────────────┐
                    │     End      │
                    └──────────────┘
```

# FIG. 4

20

# FIG. 5

(Polar Coordinate Transformed Image)                              22

# FIG. 6

(Corrected Image)                                                    24

# FIG. 7

26

# FIG. 8

# FIG. 9

EP 4 353 141 A1

# FIG. 10

(Offset Processing On)

# FIG. 11

(Offset Processing Off) Reference Example

FIG. 12

## FIG. 13A

## FIG. 13B

| | Europäisches Patentamt / European Patent Office / Office européen des brevets | **EUROPEAN SEARCH REPORT** | **Application Number** EP 23 20 2681 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 844 702 A1 (TOMEY CORP [JP]) 17 October 2007 (2007-10-17) | 1-4,7 | INV. A61B3/107 |
| Y | * paragraphs [0005] – [0039]; figures 1-12 * | 5,6 | ADD. A61B3/00 A61B3/10 |
| X | JP 2006 167002 A (TOMEY CORP) 29 June 2006 (2006-06-29) * paragraphs [0011] – [0021]; figures 1-18 * | 1-3,7 | |
| X | AU 2020 344 199 A1 (E SWIN DEV [FR]) 31 March 2022 (2022-03-31) * paragraphs [0026] – [0112]; figures 1-17 * | 1,7 | |
| X | JP 2020 010986 A (TOMEY CORP) 23 January 2020 (2020-01-23) * paragraphs [0005] – [0007], [0025] * | 1 | |
| Y | US 2014/104574 A1 (GRENON STEPHEN M [US] ET AL) 17 April 2014 (2014-04-17) * paragraph [0139]; figure 20 * | 5,6 | TECHNICAL FIELDS SEARCHED (IPC) A61B |

The present search report has been drawn up for all claims

| Place of search | Date of completion of the search | Examiner |
|---|---|---|
| Munich | 13 February 2024 | Mäki-Mantila, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 20 2681**

This annex lists the patent family members relating to the patent documents cited in the above-mentioned European search report.
The members are as contained in the European Patent Office EDP file on
The European Patent Office is in no way liable for these particulars which are merely given for the purpose of information.

**13-02-2024**

| Patent document cited in search report | | | Publication date | Patent family member(s) | | | Publication date |
|---|---|---|---|---|---|---|---|
| EP 1844702 | A1 | | 17-10-2007 | EP | 1844702 | A1 | 17-10-2007 |
| | | | | JP | 4624122 | B2 | 02-02-2011 |
| | | | | JP | 2006204773 | A | 10-08-2006 |
| | | | | US | 2008309872 | A1 | 18-12-2008 |
| | | | | WO | 2006080217 | A1 | 03-08-2006 |
| JP 2006167002 | A | | 29-06-2006 | JP | 4620443 | B2 | 26-01-2011 |
| | | | | JP | 2006167002 | A | 29-06-2006 |
| AU 2020344199 | A1 | | 31-03-2022 | AU | 2020344199 | A1 | 31-03-2022 |
| | | | | BR | 112022004443 | A2 | 31-05-2022 |
| | | | | CA | 3154503 | A1 | 18-03-2021 |
| | | | | CL | 2022000595 | A1 | 21-10-2022 |
| | | | | CN | 114727755 | A | 08-07-2022 |
| | | | | EP | 4027862 | A1 | 20-07-2022 |
| | | | | FR | 3100702 | A1 | 19-03-2021 |
| | | | | IL | 291207 | A | 01-05-2022 |
| | | | | JP | 2022548110 | A | 16-11-2022 |
| | | | | US | 2022330814 | A1 | 20-10-2022 |
| | | | | WO | 2021048510 | A1 | 18-03-2021 |
| JP 2020010986 | A | | 23-01-2020 | JP | 2020010986 | A | 23-01-2020 |
| | | | | JP | 2023101027 | A | 19-07-2023 |
| US 2014104574 | A1 | | 17-04-2014 | NONE | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004321508 A

- JP 2020010986 A